(19) Europäisches Patentamt
European Patent Office
Office européen des brevets

(11) **EP 1 958 949 A1**

(12) **EUROPEAN PATENT APPLICATION**
published in accordance with Art. 158(3) EPC

(43) Date of publication:
**20.08.2008 Bulletin 2008/34**

(21) Application number: **06834079.3**

(22) Date of filing: **06.12.2006**

(51) Int Cl.:
*C07D 409/06* (2006.01)      *A61K 9/06* (2006.01)
*A61K 9/08* (2006.01)      *A61K 31/4178* (2006.01)
*A61P 27/02* (2006.01)

(86) International application number:
**PCT/JP2006/324324**

(87) International publication number:
**WO 2007/066678 (14.06.2007 Gazette 2007/24)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **06.12.2005 JP 2005351712**

(71) Applicant: **SANTEN PHARMACEUTICAL CO., LTD.
Higashiyodogawa-ku,
Osaka-shi,
Osaka 533-8651 (JP)**

(72) Inventors:
• **SHIBAGAKI, Keiichi
Ikoma-shi, Nara 630-0101 (JP)**
• **HIRAI, Shin-ichiro
Ikoma-shi, Nara 630-0101 (JP)**
• **NAKAMURA, Masatsugu
Ikoma-shi, Nara 630-0101 (JP)**

(74) Representative: **Müller-Boré & Partner
Patentanwälte
Grafinger Strasse 2
81671 München (DE)**

(54) **THERAPEUTIC AGENT FOR CORNEAL/CONJUCTIVAL DISEASE**

(57) An object of the present invention is to discover a new use of eprosartan or a salt thereof. Eprosartan or a salt thereof exhibits an excellent improving effect in a corneal disorder model, and therefore is useful as a therapeutic agent for a keratoconjunctival disorder such as dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis and filamentary keratitis.

**EP 1 958 949 A1**

## Description

Technical Field

The present invention relates to a therapeutic agent for a keratoconjunctival disorder such as dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis, comprising eprosartan ((E)-$\alpha$-[[2-n-butyl-1-[(4-carboxyphenyl)methyl]-1H-imidazol -5-yl] methylene] -2-thiophenepropionic acid) or a salt thereof as an active ingredient.

Background Art

Cornea is a transparent avascular tissue having a diameter of about 1 cm and a thickness of about 1 mm, while conjunctiva is a mucosal membrane covering the eyeball surface posterior to the corneal margin, and the back face of the eyelid. The cornea and the conjunctiva are known to significantly affect the visual function. Keratoconjunctival disorders caused due to a variety of diseases such as corneal ulcer, keratitis, conjunctivitis, dry eyes and the like may adversely affect normal architecture of corneal epithelium, and furthermore, may impair structures and functions of the corneal stroma and corneal endothelium when the repair of these disorders is retarded, alternatively when these disorders are prolonged without making repair on some grounds. That is because the cornea and the conjunctiva are connected tissues. In these years, with the development of cell biology, factors involved in cell proliferation, migration, adhesion, extension, differentiation and the like had been elucidated, and it was reported that these factors play important roles in repair of keratoconjunctival disorders (Japanese Review of Clinical Ophthalmology, 46, 738-743 (1992), and Ophthalmic Surgery, 5, 719-727 (1992)).

On the other hand, JP-B-7-68223 discloses that eprosartan inhibits the action of angiotensin II and is useful as a therapeutic agent for hypertension, heart failure and the like.

However, there has been no report in which a pharmacological effect of eprosartan on a keratoconjunctival disorder is studied.

Disclosure of the invention

Problems to be Solved

Accordingly, it is a very interesting subject to discover a new medicinal use of eprosartan or a salt thereof.

Means for Solving the Problems

The present inventors have made intensive studies in order to discover a new medicinal use of eprosartan, and as a result, they found that eprosartan mesylate exerts an excellent improving effect on a corneal disorder in a test for a therapeutic effect using corneal disorder models and thus the present invention has been completed.

That is, the present invention is directed to a therapeutic agent for a keratoconjunctival disorder such as dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis, comprising eprosartan (hereinafter referred to as "the present compound") or a salt thereof as an active ingredient.

The salt of the present compound is not particularly limited as long as it is a pharmaceutically acceptable salt, and examples thereof include sodium salts, potassium salts, lithium salts, calcium salts, magnesium salts, salts with an inorganic acid such as hydrochloric acid, nitric acid or sulfuric acid, salts with an organic acid such as acetic acid, fumaric acid, maleic acid, succinic acid, tartaric acid, methanesulfonic acid or paratoluenesulfonic acid, and the like. Quaternary ammonium salts are also included in the salt according to the present invention. More preferred salts are methanesulfonic acid salts, sodium salts and potassium salts. When the present compound and the salt thereof are present in the form of crystals, their crystalline polymorphisms are also included in the scope of the present invention. When there are geometric isomers of the present compound, these isomers are also included in the scope of the present invention. Further, the present compound may be in the form of a hydrate or a solvate.

The keratoconjunctival disorder as used herein means the state of damaged cornea and/or conjunctiva due to various causes, and examples thereof include dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis, filamentary keratitis and the like.

The therapeutic agent for a keratoconjunctival disorder of the present invention may be administered either orally or parenterally.

Examples of the dosage form include eye drops, ophthalmic ointments, injections, tablets, capsules, granules, powders and the like. In particular, eye drops are preferred. These can be prepared using any of generally used techniques. For example, the eye drops can be prepared using a tonicity agent such as sodium chloride or concentrated glycerin, a buffer such as sodium phosphate or sodium acetate, a surfactant such as polyoxyethylene sorbitan monooleate, polyoxyl 40 stearate or polyoxyethylene hydrogenated castor oil, a stabilizer such as sodium citrate or sodium edetate, a preservative such as benzalkonium chloride or paraben as needed. The pH of the eye drops is permitted as long as it falls within the range that is acceptable as an ophthalmic preparation, but is preferably in the range of from 4 to 8.

The ophthalmic ointments can be prepared with a generally used base such as white soft paraffin or liquid paraffin. Also, oral preparations such as tablets, capsules, granules and powders can be prepared by adding an extender such as lactose, crystalline cellulose, starch or vegetable oil, a lubricant such as magnesium stearate or talc, a binder such as hydroxypropyl cellulose or polyvinyl pyrrolidone, a disintegrant such as carboxymethyl cellulose calcium or low-substituted hydroxypropylmethyl cellulose, a coating agent such as hydroxypropylmethyl cellulose, macrogol or a silicone resin, a film forming agent such as gelatin film, and the like, as needed.

The present invention also provides a method for treating a keratoconjunctival disorder comprising administering to a patient a therapeutically effective amount of eprosartan or a salt thereof.

The dose of the present compound can properly be selected depending on the symptoms, age, dosage form and the like. In the case of an eye drop, it may be instilled once to several times a day at a concentration of from 0.00001 to 10% (w/v), preferably from 0.001 to 3% (w/v). In the case of an oral preparation, it may be administered once or divided into several times at a dose of generally from 0.1 to 5000 mg per day, preferably from 1 to 1000 mg per day.

Advantage of the Invention

As will be described below, when a test for a therapeutic effect on a corneal disorder was carried out, eprosartan mesylate ((E)-$\alpha$-[[2-n-butyl-1-[(4-carboxyphenyl)methyl]-1H-imidazol-5-yl]methylene]-2-thioph enepropionic acid monomethanesulfonate) was found to exert an excellent improving effect on corneal disorder models. Therefore, eprosartan or a salt thereof is useful as a therapeutic agent for a keratoconjunctival disorder such as dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis, filamentary keratitis and the like.

Best Mode for Carrying Out the Invention

Hereinafter, results of a pharmacological test and preparation examples will be shown, however, these examples are for understanding the present invention well, and are not meant to limit the scope of the present invention.

[Pharmacological Test]

Test For Therapeutic Effect On Corneal Disorder

Using male SD rats, corneal disorder models were produced in accordance with the method of Fuj ihara et al. (Invest. Ophthalmol. Vis. Sci. 42 (1): 96-100 (2001)). After the production of the corneal disorder models, the corneal disorder score was evaluated in accordance with the method of Murakami et al. (Journal of the eye 21 (1): 87-90 (2004)), and the improvement ratio of corneal disorder after instillation of eprosartan mesylate (hereinafter referred to as "Compound A") was obtained.

(Test Method)

As test animals, male SD rats were used, and systemic anesthesia was given to the rats by administration of Nembutal. Subsequently the exorbital lacrimal gland was removed and a corneal disorder was induced over a period of 2 months.

Then, Compound A was administered as follows.

Compound A Instillation Group:

A phosphate-buffered saline solution (PBS solution) containing Compound A (0.1%) was instilled into both eyes 6 times a day for 14 days (instilled amount: 5 $\mu$L/dose) (one group consisting of 4 animals, 8 eyes).

In a control group, PBS solution was instilled into both eyes 6 times a day for 14 days (instilled amount: 5 $\mu$L/dose) (one group consisting of 4 animals, 8 eyes).

Fourteen days after the start of instillation, the damaged parts of the cornea were stained with fluorescein. For each of the upper, middle and lower parts of the cornea, the degree of fluorescein staining was evaluated by scoring according to the criteria shown below and the improvement ratio of corneal disorder was calculated from the mean value of the total scores for each of the above-mentioned parts.

Also for normal eyes, the mean value of the total scores for each of the above-mentioned parts was obtained.

(Evaluation Criteria)

0: No punctate staining
1: Scattered staining (punctate, separated staining)
2: Moderate staining (a part of punctate staining being adjacent)
3: Heavy staining (punctate, barely separated staining)

(Results)

By taking the mean value of the total scores for the control group (PBS solution) as a standard (improvement ratio: 0%) and according to the calculation formula shown below, the improvement ratio in the Compound A instillation group was calculated, which is shown in Table 1. Incidentally, the mean value of the scores is a mean of those of 8 cases, respectively.

$$\text{Improvement ratio (\%)} = \{(\text{control}) - (\text{Compound A})\} / \text{damage degree} \times 100$$

$$\text{Damage degree} = (\text{control}) - (\text{normal eye})$$

[Table 1]

|  | Mean value of scores | Improvement ratio (%) |
|---|---|---|
| Normal eye | 2.8 |  |
| Control group | 6.1 |  |
| Compound A instillation group (0.1%) | 3.3 | 84.8 |

(Discussion)

As apparent from the results of the above pharmacological test using rats (Table 1), Compound A significantly improved a corneal damage.

[Preparation Examples]

Hereinafter, representative preparation examples using Compound A will be shown.

Formulation example 1

In 100 ml,

Compound A          10 mg
Sodium Chloride     900 mg
Sterile purified water   q.s.

By altering the amount of Compound A to be added, an eye drop at a concentration of 0.001% (w/v) , 0.03% (w/v) , 0.1% (w/v), 0.3% (w/v), 1.0% (w/v), or 3.0% (w/v) can be prepared.

Formulation example 2

In 100 g,

| | |
|---|---|
| Compound A | 0.3 g |
| Liquid paraffin | 10.0 g |
| White soft paraffin | q.s. |

By altering the amount of Compound A to be added, an ophthalmic ointment at a concentration of 1% (w/w) or 3% (w/w) can be prepared.

Industrial Applicability

Eprosartan or a salt thereof is useful as a therapeutic agent for a keratoconjunctival disorder such as dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis and filamentary keratitis.

**Claims**

1.    A therapeutic agent for a keratoconjunctival disorder, comprising eprosartan or a salt thereof as an active ingredient.

2.    The therapeutic agent for a keratoconjunctival disorder according to claim 1, wherein the keratoconjunctival disorder is dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis.

3.    The therapeutic agent for a keratoconjunctival disorder according to claim 1 or 2, wherein the dosage form of the agent is an eye drop or an ophthalmic ointment.

4.    A method for treating a keratoconjunctival disorder comprising administering to a patient a therapeutically effective amount of eprosartan or a salt thereof.

5.    The method for treating a keratoconjunctival disorder according to claim 4, wherein the keratoconjunctival disorder is dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis.

6.    The method for treating a keratoconjunctival disorder according to claim 4 or 5, wherein the administration form of eprosartan or a salt thereof is an eye drop or an ophthalmic ointment.

7.    Use of eprosartan or a salt thereof for manufacturing a therapeutic agent for a keratoconjunctival disorder.

8.    The use according to claim 7, wherein the keratoconjunctival disorder is dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis.

9.    The use according to claim 7 or 8, wherein the dosage form of the agent is an eye drop or an ophthalmic ointment.

## INTERNATIONAL SEARCH REPORT

| | International application No. |
|---|---|
| | PCT/JP2006/324324 |

A. CLASSIFICATION OF SUBJECT MATTER
*C07D409/06*(2006.01)i, *A61K9/06*(2006.01)i, *A61K9/08*(2006.01)i, *A61K31/4178*
(2006.01)i, *A61P27/02*(2006.01)i

According to International Patent Classification (IPC) or to both national classification and IPC

B. FIELDS SEARCHED

Minimum documentation searched (classification system followed by classification symbols)
C07D409/06, A61K9/06, A61K9/08, A61K31/4178, A61P27/02

Documentation searched other than minimum documentation to the extent that such documents are included in the fields searched

| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS(STN), CAplus(STN), EMBASE(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| Y | JP 07-053408 A (HOFFMANN LA ROCHE), 28 February, 1995 (28.02.95), Particularly, page 2, left column, lines 17 to 36; page 5, left column, line 31 to page 6, left column, line 19 & EP 634175 A1 & US 5696116 A | 1-3,7-9 |
| Y | JP 10-218792 A (SANTEN PHARM CO., LTD.), 18 August, 1998 (18.08.98), Particularly, Claims; page 3, left column, line 27 to right column, line 45 (Family: none) | 1-3,7-9 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 25 January, 2007 (25.01.07) | 06 February, 2007 (06.02.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

INTERNATIONAL SEARCH REPORT

International application No.

PCT/JP2006/324324

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2001-058958 A (SENJU SEIYAKU KABUSHIKI KAISHA), 06 March, 2001 (06.03.01), Particularly, Claims; page 2, left column, lines 28 to 31, right column, lines 27 to 37; page 16, right column, lines 14 to 24 (Family: none) | 1-3,7-9 |
| Y | JP 2001-031636 A (SENJU SEIYAKU KABUSHIKI KAISHA), 06 February, 2001 (06.02.01), Particularly, Claims; page 2, left column, line 45 to right column, line 11; page 14, left column, line 4 to right column, line 10 (Family: none) | 1-3,7-9 |
| A | CHESNOKOVA, N.B. et al, The role of the components of therenin-angiotensin system in the ocular tissues in norm andpathology, Vestn Ross Akad Med Nauk, 2003, Vol.9, p.29-32 | 1-3,7-9 |

Form PCT/ISA/210 (continuation of second sheet) (April 2005)

**INTERNATIONAL SEARCH REPORT**

International application No.

PCT/JP2006/324324

| **Box No. II** | **Observations where certain claims were found unsearchable (Continuation of item 2 of first sheet)** |
|---|---|

This international search report has not been established in respect of certain claims under Article 17(2)(a) for the following reasons:

1. ☒ Claims Nos.: 4-6

because they relate to subject matter not required to be searched by this Authority, namely:
Claims 4 to 6 pertain to methods for treatment of the human body by therapy and thus relate to a subject matter which this International Searching Authority is not required, under the provisions of the PCT Rule 39.1(iv), to search.

2. ☐ Claims Nos.:

because they relate to parts of the international application that do not comply with the prescribed requirements to such an extent that no meaningful international search can be carried out, specifically:

3. ☐ Claims Nos.:

because they are dependent claims and are not drafted in accordance with the second and third sentences of Rule 6.4(a).

| **Box No. III** | **Observations where unity of invention is lacking (Continuation of item 3 of first sheet)** |
|---|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7068223 B

**Non-patent literature cited in the description**

- *Japanese Review of Clinical Ophthalmology,* 1992, vol. 46, 738-743
- *Ophthalmic Surgery,* 1992, vol. 5, 719-727
- **FUJIHARA et al.** *Invest. Ophthalmol. Vis. Sci.,* 2001, vol. 42 (1), 96-100
- **MURAKAMI et al.** *Journal of the eye,* 2004, vol. 21 (1), 87-90